# FASCICULE DE BREVET EUROPEEN

(11) **EP 1 276 743 B1**
(45) Date de publication et mention de la délivrance du brevet: **09.08.2006**
(21) Numéro de dépôt: 01929692.0
(22) Date de dépôt: 24.04.2001
(51) Int. Cl.: C07F 7/00, C07F 9/00, C07F 7/22, C07F 9/90, C07F 9/6568

(54) **LIQUIDES IONIQUES DERIVES D'ACIDES DE LEWIS A BASE DE TITANE, NIOBIUM, TANTALE OU ANTIMOINE, ET LEURS APPLICATIONS**
IONISCHE FLÜSSIGKEITEN ABGELEITET VON LEWISSÄURE AUF DER BASIS VON TITAN,NIOBIUM,TANTAL ODER ANTIMON, UND DEREN VERWENDUNGEN
ION LIQUIDS DERIVED FROM LEWIS ACID BASED ON TITANIUM, NIOBIUM, TANTALUM OR ANTIMONY, AND USES THEREOF

(30) Priorité: 26.04.2000 FR 0005275
(43) Date de publication de la demande: 22.01.2003
(73) Titulaire: Arkema France, 92800 Puteaux (FR)
(72) Inventeur: BONNET, Philippe, F-69007 Lyon (FR); LACROIX, Eric, F-69480 Ambérieux d'Azergues (FR); SCHIRMANN, Jean-Pierre, F-75011 Paris (FR)
(86) Numéro de dépôt international: PCT/FR2001/001245
(87) Numéro de publication internationale: WO 2001/081353

(56) Documents cités:
- WO-A-00/20115
- WO-A-00/56700
- WO-A-99/40025
- FR-A- 2 611 700

## Description

La présente invention concerne le domaine des liquides ioniques et a plus particulièrement pour objet des liquides ioniques à base de titane, de niobium, de tantale, d'étain ou d'antimoine.

Les liquides ioniques tels que considérés dans la présente demande de brevet sont des sels non aqueux à caractère ionique qui sont liquides à des températures modérées et résultent de la réaction entre un sel organique et un composé inorganique.

On connaît déjà un certain nombre de liquides ioniques. Ainsi, dans J.Am. Chem. Soc. 101:2, 323-327 (1979), Osteryoung et al. ont mis en évidence que le mélange de chlorure de butylpyridinium (A) et de chlorure d'aluminium AlCl₃ (B) est liquide à 40°C sur une large plage de compositions (rapport molaire A/B allant de 1:0,75 à 1:2. Dans Inorg, Chem. 21, 1263-1264, Wilkes et al. ont montré que les composés issus de la réaction entre AlCl₃ et un chlorure de dialkylimidazolium tel que le chlorure de 1-butyl-3-méthyl-imidazolium sont liquides à température ambiante également pour la même plage de compositions décrite précédemment. Dans cet exemple de liquide ionique, le dialkyl-imidazolium constitue le cation et AlCl₄ l'anion. D'autres anions tels que les anions nitrate, acétate ou tétrafluoroborate peuvent être utilisés (voir la publication WO 9618459).

En raison de leur fenêtre électrochimique étendue, les liquides ioniques peuvent être utilisés comme électrolytes de batterie. Ils sont également particulièrement utiles pour la catalyse car ce sont d'excellents solvants de composés organométalliques. Ainsi, par exemple, les demandes de brevet FR 2 626 572 et WO 9521806 décrivent l'emploi des composés cités précédemment à base de chloroaluminate pour l'alkylation en catalyse biphasique de composés aromatiques ou d'isoparaffines.

Le document WO 00/56700 divulgue un composé ionique ayant une température de fusion au plus égale à 60°C et obtenu par réaction d'un ammonium quaternaire avec un halogénure de Zn, Sn ou Fe.

Le document WO 00/20115 divulgue un sel ionique comprenant au moins un cation organique et un anion de formule [BX₃Y]⁻ avec X représentant un atome d'halogène et Y est choisi parmi un halogène autre que X, une fonction éther et carboxylique.

Le document FR 2611700 divulgue un mélange liquide ionique constitué d'halogénure d'aluminium et d'au moins un halogénure d'ammonium quaternaire.

Le document WO 99/40025 divulgue un composé ionique de bas point de fusion dont le cation est de type onium possédant au moins un hétéroatome tel que N, O, S ou P portant la charge positive et dont l'anion inclut au moins un ion imidure.

L'invention a mainten pour objet des liquides ioniques dérivés d'acides de Lewis à base de titane, de niobium, de tantale ou d'antimoine. Ces liquides ioniques trouvent des applications en catalyse et peuvent également avoir des propriétés solvantes de composés organométalliques susceptibles d'être utilisés en catalyse biphasique.

Les liquides ioniques selon l'invention qui sont des composés ioniques non aqueux, aprotiques et liquides dans une gamme de températures modérées (de préférence en-dessous de 120°C) à pression atmosphérique, sont obtenus par réaction d'au moins un acide de Lewis halogéné ou oxyhalogéné à base de titane, de niobium, de tantale ou d'antimoine avec un sel de formule générale X⁺A⁻, dans laquelle A⁻ désigne un anion halogénure (bromure, iodure et, de préférence chlorure ou fluorure) ou hexafluoroantimoniate (SbF₆⁻) et X⁺ un cation ammonium quaternaire, phosphonium quaternaire ou sulfonium ternaire.

L'acide de Lewis halogéné à base de titane, de niobium, de tantale ou d'antimoine peut être un dérivé chloré, bromé, fluoré ou mixte, par exemple un acide chlorofluoré. Peuvent être mentionnés plus particulièrement les chlorures, fluorures ou chlorofluorures des formules suivantes :
TiClₓF_{y} avec x+y = 4 et 0 ≤ x ≤ 4
TaClₓF_{y} avec x+y = 5 et 0 ≤ x ≤ 5
NbClₓF_{y} avec x+y = 5 et 0 ≤ x ≤ 5
SbClₓF_{y} avec x+y = 5 et 0 ≤ x ≤ 5

Comme exemples de tels composés, on peut mentionner les composés suivants : TiCl₄, TiF₄, TaCl₅, TaF₅, NbCl₅, NbF₅, SbCl₅, SbCl₄F, SbCl₃F₂, SbCl₂F₃, SbClF₄, SbF₅ et leurs mélanges. Sont préférentiellement utilisés les composés suivants : TiCl₄, TaCl₅+TaF₅, NbCl₅+NbF₅, SbCl₅, SbFCl₄, SbF₂Cl₃, SbF₃Cl₂, SbF₄Cl, SbF₅ et SbCl₅+SbF₅. Sont plus particulièrement préférés les composés antimoniés.

Comme exemples d'acides de Lewis oxyhalogénés, utilisables selon l'invention, on peut mentionner TiOCl₂, TiOF₂et SbOClₓF_{y} (x+y=3).

Dans le sel X⁺A⁻, le cation X⁺ peut répondre à l'une des formules générales suivantes:
R¹R²R³R⁴N⁺
R¹R²R³R⁴P⁺
R¹R²R³S⁺
dans lesquelles les symboles R¹ à R⁴, identiques ou différents, désignent chacun un groupement hydrocarbyle, chlorohydrocarbyle, fluorohydrocarbyle, chlorofluorohydrocarbyle ou fluorocarbyle ayant de 1 à 10 atomes de carbone, saturé ou non, cyclique ou non, ou aromatique, l'un ou plusieurs de ces groupements pouvant également contenir un ou plusieurs hétéroatomes tels que N, P, S ou O.

Le cation ammonium, phosphonium ou sulfonium X⁺ peut également faire partie d'un hétérocycle saturé ou non, ou aromatique ayant de 1 à 3 atomes d'azote, de phosphore ou de soufre, et répondre à l'une ou l'autre des formules générales suivantes : 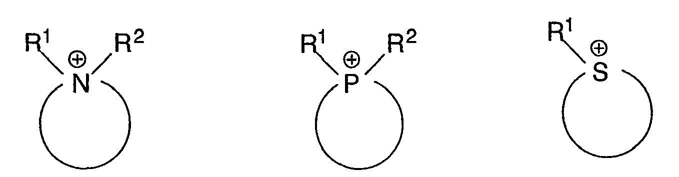 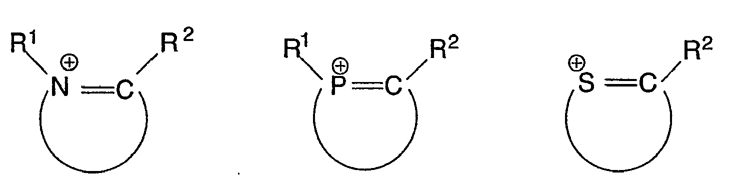 dans lesquelles R¹ et R² sont tels que définis précédemment.

On ne sortirait pas du cadre de la présente invention en utilisant un sel contenant 2 ou 3 sites ammonium, phosphonium ou sulfonium dans leur formule.

Comme exemples de sels X⁺A⁻ on peut mentionner les chlorures et fluorures de tétraalkyl ammonium, les chlorures et fluorures de tétraalkyl phosphonium, et chlorures et fluorures de trialkyl sulfonium, les chlorures et fluorures d'alkyl pyridinium, les chlorures, fluorures et bromures de dialkyl imidazolium, et les chlorures et fluorures de trialkyl imidazolium. Sont plus particulièrement appréciés le fluorure ou le chlorure de triméthyl sulfonium, le chlorure ou le fluorure de N-éthyl-pyridinium, le chlorure ou le fluorure de N-butyl-pyridinium, le chlorure ou le fluorure de 1-éthyl-3-méthyl-imidazolium, et le chlorure ou fluorure de 1-butyl-3-méthyl-imidazolium.

Les liquides ioniques selon l'invention peuvent être préparés de façon connue en soi en mélangeant de manière appropriée l'acide de Lewis halogéné ou oxyhalogéné et le sel organique X⁺A⁻ dans un rapport molaire qui peut aller de 0,5:1 à 3,5:1, de préférence de 1:1 à 2,5:1 et plus préférentiellement 1:1 à 2:1. Un rapport molaire strictement supérieur à 1:1 est particulièrement recommandé si l'on désire obtenir un liquide ionique acide.

Le mélange peut être réalisé dans un réacteur de type autoclave, éventuellement refroidi pour limiter l'exothermie de la réaction. On peut également contrôler cette exothermie en ajoutant progressivement l'un des réactifs à l'autre.

Lorsque le rapport molaire acide de Lewis/sel organique est supérieur à 1:1, il peut s'avérer utile de chauffer le mélange réactionnel jusqu'à dissolution complète du solide.

Les réactifs et le liquide ionique obtenu étant généralement hygroscopiques, il est recommandé de réaliser la synthèse à l'abri de l'air et de l'eau, en utilisant à cet effet les moyens connus de l'homme de l'art.

Les liquides ioniques selon l'invention peuvent être utilisés dans toutes les applications connues pour ce type de composés. Quand le rapport molaire acide de Lewis/sel organique est inférieur ou égal à 1:1, ces liquides pourront être utilisés pour leurs propriétés solvantes (synthèse organique, solvants pour complexes organométalliques).

Quand le rapport molaire acide de Lewis/sel organique est strictement supérieur à 1:1, les liquides ioniques selon l'invention ont des propriétés acides intéressantes pour toute catalyse requérant l'emploi d'un catalyseur acide, par exemple la catalyse de Friedel et Crafts et la catalyse de fluoration.

Les liquides ioniques selon l'invention trouvent une utilisation particulièrement intéressante pour la fluoration par HF en phase liquide de composés saturés ou insaturés présentant des liaisons C-Cl, tels que par exemple CHCl=CH₂, CCl₂=CCl₂, CH₂=CCl₂, CClH=CCl₂, CH₂Cl₂, CH₂ClF, CCl₃-CH₃, CCl₂H-CCl₂H, CCl₃-CH₂Cl, CCl₃₋CHCl₂, CF₃-CH₂Cl, CCl₃-CH₂-CHCl₂, CF₃-CH=CHCl, CCl₃-CH₂-CCl₂-CH₃.

Un procédé de fluoration en phase liquide utilisant comme catalyseur un liquide ionique selon l'invention peut être mis en oeuvre en discontinu. L'appareillage utilisé est alors un autoclave dans lequel les réactifs et le catalyseur sont introduits avant le début de la réaction, la pression dans l'autoclave variant avec l'avancement de la réaction. La quantité molaire d'HF sur la quantité molaire de produit de départ est comprise entre 2 et 50 et préférentiellement entre 10 et 30. La durée de la réaction nécessaire qui dépend de la quantité des réactifs mise en jeu au départ et des différents paramètres opératoires, peut être facilement connue expérimentalement.

La fluoration peut également être mise en oeuvre selon un mode semi-continu dans un appareillage constitué d'un autoclave surmonté d'un condenseur simple, ou d'une colonne de rétrogradation avec un condenseur de reflux, et d'une vanne de régulation de pression, la pression étant choisie de façon à maintenir le milieu réactionnel à l'état liquide. Comme précédemment, les réactifs et le catalyseur sont préalablement introduits dans le réacteur, mais les produits de la réaction à bas point d'ébullition (produits fluorés) et le chlorure d'hydrogène (HCl) co-produit sont extraits en continu durant la réaction, tandis que les composés à plus haut point d'ébullition tels que les produits de départ, les produits intermédiaires et l'HF sont reflués en majeure partie sous forme liquide dans le milieu réactionnel grâce au condenseur (ou colonne de rétrogradation) placé au-dessus du réacteur.

Conformément à un troisième mode de réalisation, la fluoration peut être conduite en continu dans le même appareillage qu'en semi-continu, mais l'un au moins des réactifs (HF ou le produit de départ) est introduit en continu. Dans le cadre d'un procédé industriel, on préfère l'alimentation conjointe des deux réactifs. Les produits de la réaction sont extraits en continu pendant la réaction, les réactifs non transformés étant reflués au réacteur. La quantité molaire d'HF alimenté sur la quantité molaire de produit de départ alimenté est au moins égale à 2.

La quantité de catalyseur dépend des conditions opératoires, du milieu réactionnel (dans le cas d'un procédé en continu) mais également de l'activité intrinsèque du catalyseur. Cette quantité est comprise entre 0.5 et 90% (molaire) du milieu réactionnel.

Lorsque le catalyseur utilisé est à base d'antimoine, il peut parfois être avantageux d'introduire du chlore pour maintenir l'antimoine au degré d'oxydation 5.

La température à laquelle est réalisée la réaction de fluoration (en conditions discontinues et continues) est généralement comprise entre 30 et 180°C, de préférence entre 80 et 130°C.

La pression à laquelle la réaction est réalisée en modes semi-continu ou continu est choisie de manière à maintenir le milieu réactionnel en phase liquide. Elle se situe généralement entre 5 et 50 bars et préférentiellement entre 10 et 40 bars; en conditions continues, si HF constitue le milieu réactionnel, la pression de fonctionnement choisie est en général la pression de vapeur saturante de l'HF à la température de réaction désirée. La température du condenseur est fixée en fonction de la quantité et de la nature des produits susceptibles d'être évacués durant la réaction. Elle est généralement comprise entre -50 et 150°C et préférentiellement entre 0 et 100°C.

Le matériau du réacteur de fluoration doit permettre de travailler dans les conditions de pression et de température définies précédemment. Il doit également résister à la corrosion qu'engendre le fluorure d'hydrogène. Ainsi, l'acier inoxydable ou des alliages type MONEL, INCONEL ou HASTELLOY sont particulièrement indiqués.

Les exemples suivants illustrent l'invention sans la limiter.

### EXEMPLE 1

Dans un tube de Schlenk on a introduit sous balayage d'argon, 7,3 g de chlorure de 1-éthyl-3-méthyl-imidazolium (emim-Cl), puis le tube a été plongé dans de la glace. On a alors introduit progressivement, sous agitation, du chlorofluorure d'antimoine SbF₃Cl₂. La réaction est exothermique mais la glace a permis de maintenir la température du tube vers 10°C. Après avoir introduit 12,6 g de SbF₃Cl₂ correspondant à un ratio molaire SbF₃Cl₂/emim-Cl de 1:1, le tube de Schlenk a été chauffé vers 40°C afin de parfaitement liquéfier le liquide ionique formé. On a ensuite continué d'ajouter du SbF₃Cl₂ à température ambiante jusqu'à une masse totale introduite de 25,3 g correspondant à un ratio molaire SbF₃Cl₂/emim-Cl de 2:1.

Après 18 heures d'agitation à température ambiante, on a obtenu 32,6 g d'un liquide ionique. Ce composé a été analysé par spectroscopie Infra Rouge et Raman et présente les caractéristiques principales suivantes :
**IR**
   - bande à 1600 cm⁻¹ (attribuable à la liaison C=N)
   - bande entre 550 et 600 cm⁻¹
**Raman**
   - bande à 370 cm⁻¹
   - bande à 174 cm⁻¹

### EXEMPLE 2

On a répété l'exemple 1, mais en remplaçant, d'une part, le chlorofluorure d'antimoine par 31,7 g d'un mélange équimolaire de pentachlorure de tantale et de pentafluorure de tantale et, d'autre part, l'emim-Cl par 8,7 g de chlorure de 1-butyl-3-méthyl-imidazolium (bmim-Cl).

### EXEMPLES 3 A 14

Le tableau suivant résume d'autres exemples de liquides ioniques selon l'invention obtenus de la même façon qu'aux exemples 1 et 2. Lorsqu'un liquide n'est pas rapidement obtenu, le mélange d'acide de Lewis et d'emim-Cl est chauffé à 120°C pendant 12 heures en plongeant le tube de Schlenk dans un bain d'huile à 120°C. Tous les produits obtenus sont liquides en dessous de 120°C.

| **Exemple** | **Acide de Lewis** | **Rapport molaire** | **Caractéristique** |
|---|---|---|---|
| | | **Acide de Lewis/emim-Cl** | **(% massique de métal** |
| | | | **analysé)** |
| 3 | TiCl₄ | 1:1 | 14 |
| 4 | TiCl₄ | 2:1 | 18 |
| 5 | SnCl₄ | 1:1 | 29 |
| 6 | SbF₄Cl | 1:1 | 31 |
| 7 | SbF₄Cl | 2:1 | 39 |
| 8 | NbCl₅ | 1:1 | 22 |
| 9 | NbF₅ | 1:1 | 29 |
| 10 | NbCl₅+ NbF₅ ^{*} | 2:1 | 31 |
| 11 | SbF₅ | 1:1 | 33 |
| 12 | SbF₅ | 2:1 | 43 |
| 13 | SbCl₅ | 1:1 | 27 |
| 14 | SbCl₅ | 2:1 | 33 |

| | | | |
|---|---|---|---|
| ^{*}mélange équimolaire de NbCl₅ et NbF₅ | | | |

### EXEMPLES 15 A 24

Le tableau suivant résume d'autres exemples de liquides ioniques selon l'invention obtenus de la même façon qu'aux exemples 1 et 2, mais en utilisant le chlorure de 1-butyl-3-méthyl-imidazolium (bmim-Cl) comme sel d'ammonium quaternaire. Lorsqu'un liquide n'est pas rapidement obtenu, le mélange d'acide de Lewis et de bmim-Cl est chauffé à 120°C pendant 12 heures en plongeant le tube de Schlenk dans un bain d'huile à 120°C. Les produits obtenus sont tous liquides en dessous de 120°C.

| **Exemple** | **Acide de Lewis** | **Rapport molaire** | **Caractéristique** |
|---|---|---|---|
| | | **acide de Lewis/bmim-Cl** | **(% massique de métal** |
| | | | **analysé)** |
| 15 | TiCl₄ | 1:1 | 13 |
| 16 | TiCl₄ | 2:1 | 17 |
| 17 | TiF₄ | 1:1 | 16 |
| 18 | SnCl₄ | 1:1 | 27 |
| 19 | NbCl₅ | 1:1 | 21 |
| 20 | TaCl₅ | 1:1 | 34 |
| 21 | SbCl₅ | 1:1 | 26 |
| 22 | SbCl₅ | 2:1 | 31 |
| 23 | SbF₅ | 1:1 | 31 |
| 24 | SbF₅ | 2:1 | 40 |

### EXEMPLE 25

Dans un tube de Schlenk contenant 7,3 g d'emim-Cl maintenu en suspension dans 30 ml d'acétone sous forte agitation, on a ajouté 12,9 g d'hexafluoroantimoniate de sodium. Il s'est formé instantanément un précipité laiteux qui, après 18 heures d'agitation, a été filtré sur Celite® . Après avoir évaporé l'acétone sous vide, on a obtenu un liquide translucide auquel, le tube de Schlenk ayant été plongé dans de la glace pour le refroidir, on a ajouté 10,8 g de pentafluorure d'antimoine, puis laissé revenir le mélange à la température ambiante.

### EXEMPLE 26

Dans un autoclave en inox 316L de 0,150 litre ont été successivement chargés 30,6 g du liquide ionique de l'exemple 7, 21,5 g de chlorure de méthylène (F30) et 20,8 g d'HF. L'autoclave a alors été plongé dans un bain d'huile à 130°C, la température du condenseur étant maintenue à 15-20°C et la pression de régulation fixée à 10 bars absolus. A cette pression, la température du milieu réactionnel était d'environ 100°C.

Durant la réaction, les produits de la réaction volatils étaient évacués en continu et passaient dans un barboteur à eau, puis dans un sécheur avant d'être recueillis dans un piège en inox refroidi à l'azote liquide. Après 290 minutes de réaction, l'autoclave a été mis en refroidissement par circulation d'eau. Après retour à température ambiante, l'autoclave a été dégazé et les produits de la réaction ont été lavés, séchés et piégés comme précédemment.

L'analyse de la phase gaz et de la phase liquide des différents pièges a montré que 84 % du F30 initial a été converti, dont 69 % en difluorométhane et 31 % en chlorofluorométhane (% molaires).

### EXEMPLE 27

Dans un autoclave en inox 316L de 1 litre, équipé d'un agitateur magnétique, surmonté d'un condenseur simple et asservi à une vanne de régulation de pression placée en tête du condenseur et par laquelle s'évacuent les produits de la réaction, on a chargé successivement 740 g de trichloréthylène et 102 g du liquide ionique de l'exemple 12. Par l'intermédiaire d'un bain d'huile, on a alimenté la double paroi de. l'autoclave pour obtenir une température de 125°C dans l'autoclave et on a maintenu la température du condenseur à 55°C par alimentation d'eau. Lorsque la température désirée a été atteinte, on a alimenté en continu 42 g/h de trichloréthylène, 27 g/h d'HF et 3 g/h de chlore. Pendant ce essai, un suivi permanent de l'ouverture de la vanne de régulation de pression, représentatif du débit de gaz en sortie du réacteur, a été réalisé.

Après une période de stabilisation de 14 heures correspondant à l'obtention d'une composition d'équilibre du milieu réactionnel dans l'autoclave, l'ouverture de la vanne de régulation est devenue stable. La composition des gaz en sortie de réacteur s'est alors établie à 95 % de F133a (CF₃-CH₂Cl) et 5 % de F132b (CF₂Cl-CH₂Cl).

Après 25 heures de marche stable, l'alimentation en Cl₂ a été coupée. Le tableau suivant indique les valeurs de l'ouverture de la vanne à partir de la coupure d'alimentation en Cl₂. Il apparaît clairement qu'au bout de 20 heures, l'ouverture de la vanne de régulation n'a pas varié ce qui correspond à une marche parfaitement stable.

### EXEMPLE 28 comparatif

On reproduit l'exemple précédent en chargeant 749 g de trichloréthylène et 53,2 g de SbCl₅ à la place du liquide ionique utilisé précédemment. Après obtention d'une marche stable, la température dans l'autoclave s'est établie à 125 °C.

Après 25 heures de marche stable, l'alimentation en chlore a été coupée. Les valeurs d'ouverture de la vanne de régulation de pression à partir de la coupure d'alimentation en Cl₂ sont indiquées dans le tableau suivant. On voit qu'après environ 4 heures de réaction, la vanne s'est fermée ce qui correspond à un arrêt de la réaction.

| | Valeurs d'ouverture en % de la vanne de régulation de pression après l'arrêt de l'alimentation en Cl₂ | |
|---|---|---|
| Temps (heures) | Exemple 27 | Exemple 28 comparatif |
| 1 | 10 | 13 |
| 2 | 11 | 13 |
| 3 | 10 | 11 |
| 4 | 10 | 3 |
| 5 | 10 | 0 |
| 7 | 10 | 0 |
| 70 | 12 | 0 |
| 15 | 12 | 0 |
| 20 | 10 | 0 |

## Revendications

1. Liquide ionique constitué par le produit de la réaction d'au moins un acide de Lewis halogéné ou oxyhalogéné à base de titane, de niobium, de tantale ou d'antimoine avec un sel organique de formule générale X⁺A⁻ dans laquelle A⁻ est un anion halogénure ou hexafluoroantimoniate et X⁺ un cation ammonium quaternaire, phosphonium quaternaire ou sulfonium ternaire.

2. Liquide ionique selon la revendication 1 dans lequel le rapport molaire acide de Lewis/sel organique est compris entre 0,5:1 et 3,5:1, de préférence entre 1:1 et 2,5:1 et plus particulièrement entre 1:1 et 2:1.

3. Liquide ionique selon la revendication 1 ou 2 dans lequel l'acide de Lewis est un chlorure, un fluorure ou un chlorofluorure de titane, de niobium, de tantale ou d'antimoine.

4. Liquide ionique selon la revendication 3 dans lequel l'acide de Lewis est un composé antimonié.

5. Liquide ionique selon l'une des revendications 1 à 4 dans lequel le sel organique X⁺A⁻ est un chlorure ou un fluorure, le cation X⁺ répondant à l'une des formules générales suivantes :
R¹R²R³R⁴N⁺
R¹R²R³R⁴P⁺
R¹R²R³S⁺
dans lesquelles les symboles R¹ à R⁴, identiques ou différents, désignent chacun un groupement hydrocarbyle, chlorohydrocarbyle, fluorohydrocarbyle, chlorofluorohydrocarbyle ou fluorocarbyle ayant de 1 à 10 atomes de carbone, saturé ou non, cyclique ou non, ou aromatique, l'un ou plusieurs de ces groupements pouvant également contenir un ou plusieurs hétéroatomes.

6. Liquide ionique selon l'une des revendications 1 à 4 dans lequel le sel organique X⁺A⁻ est un chlorure ou un fluorure, le cation X⁺ faisant partie d'un hétérocycle saturé ou non, ou aromatique ayant de 1 à 3 atomes d'azote, de phosphore ou de soufre, et répondant à l'une ou l'autre des formules générales suivantes : 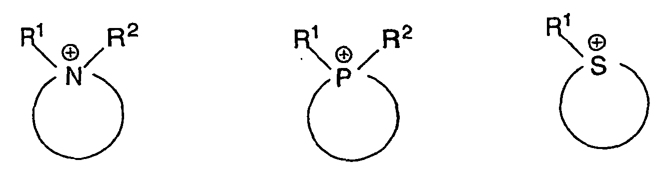 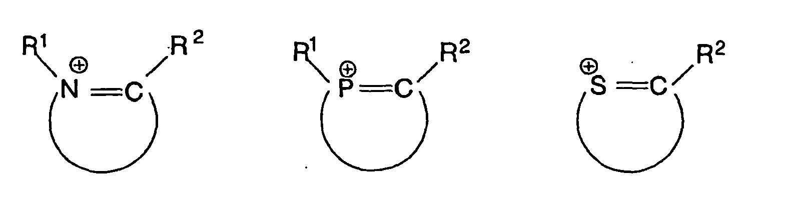 dans lesquelles R¹ et R² sont tels que définis dans la revendication 5.

7. Liquide ionique selon la revendication 5 ou 6 dans lequel le cation X⁺ est un cation tétraalkylammonium, tétraalkylphosphonium, trialkylsulfonium, alkylpyridinium, dialkylimidazolium ou trialkylimidazolium, de préférence un cation triméthylsulfonium, N-éthyl-pyridinium, N-butyl-pyridinium, 1-éthyl-3-méthyl-imidazolium ou 1-butyl-3-méthylimidazolium.

8. Procédé de fluoration par HF en phase liquide d'un composé saturé ou insaturé présentant des liaisons C-Cl, **caractérisé en ce que** l'on utilise comme catalyseur un liquide ionique selon l'une des revendications 1 à 7.

## Claims

1. Ionic liquid composed of the reaction product of at least one halogenated or oxyhalogenated Lewis acid based on titanium, niobium, tantalum or antimony with an organic salt of general formula X⁺A⁻ in which A⁻ is a halide or hexafluoroantimonate anion and X⁺ a quaternary ammonium, quaternary phosphonium or ternary sulfonium cation.

2. Ionic liquid according to Claim 1, in which the Lewis acid/organic salt molar ratio is between 0.5/1 and 3.5/1, preferably between 1/1 and 2.5/1 and more particularly between 1/1 and 2/1.

3. Ionic liquid according to Claim 1 or 2,
in which the Lewis acid is a titanium, niobium, tantalum or antimony chloride, fluoride or chlorofluoride.

4. Ionic liquid according to Claim 3, in which the Lewis acid is an antimony-comprising compound.

5. Ionic liquid according to one of
Claims 1 to 4, in which the organic salt X⁺A⁻ is a chloride or a fluoride, the cation X⁺ corresponding to one of the following general formulae:
R¹R²R³R⁴N⁺
R¹R²R³R⁴P⁺
R¹R²R³S⁺
in which the symbols R¹ to R⁴, which are identical or different, each denote a saturated or unsaturated, cyclic or noncyclic or aromatic, hydrocarbyl, chlorohydrocarbyl, fluorohydrocarbyl, chlorofluorohydrocarbyl or fluorocarbyl group having from 1 to 10 carbon atoms, it being possible for one or more of these groups also to comprise one or more heteroatoms.

6. Ionic liquid according to one of
Claims 1 to 4, in which the organic salt X⁺A⁻ is a chloride or a fluoride, the cation X⁺ forming part of a saturated or unsaturated or aromatic heterocycle having from 1 to 3 nitrogen, phosphorus or sulfur atoms and corresponding to one or other of the following general formulae: 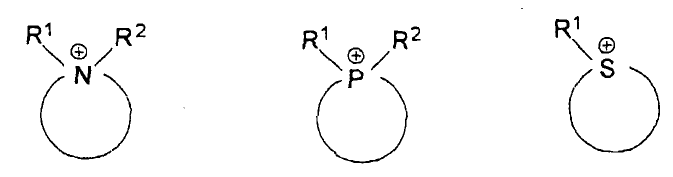 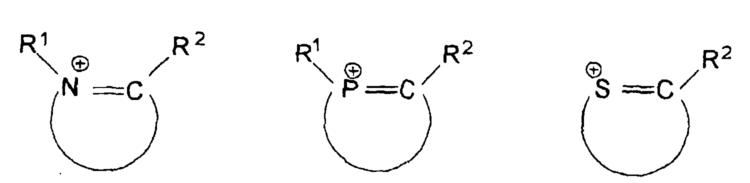 in which R¹ and R² are as defined in Claim 5.

7. Ionic liquid according to Claim 5 or 6,
in which the cation X⁺ is a tetraalkylammonium, tetraalkylphosphonium, trialkylsulfonium, alkylpyridinium, dialkylimidazolium or trialkylimidazolium cation, preferably a trimethylsulfonium, N-ethylpyridinium, N-butylpyridinium, 1-ethyl-3-methylimidazolium or 1-butyl-3-methylimidazolium cation.

8. Process for the liquid-phase fluorination by HF of a saturated or unsaturated compound exhibiting C-C1 bonds, **characterized in that** an ionic liquid according to one of Claims 1 to 7 is used as catalyst.

## Patentansprüche

1. Ionische Flüssigkeit, die besteht aus dem Produkt der Reaktion mindestens einer halogenierten oder oxyhalogenierten Lewis-Säure auf der Basis von Titan, Niob, Tantal oder Antimon mit einem organischen Salz der allgemeinen Formel X⁺A-, in der A-ein Halogenid- oder Hexafluorantimonat-Anion und X⁺ ein quaternäres Ammonium- oder Phosphonium-Kation oder ein ternäres Sulfonium-Kation bedeuten.

2. Ionische Flüssigkeit nach Anspruch 1, bei der das Molverhältnis Lewis-Säure/organisches Salz im Bereich von 0,5:1 1 bis 3,5:1, bevorzugt im Bereich von 1:1 1 bis 2,5:1 1 und ganz besonders im Bereich von 1:1 1 bis 2:1 liegt.

3. Ionische Flüssigkeit nach Anspruch 1 oder 2, bei der die Lewis-Säure ein Chlorid, ein Fluorid oder ein Chlorfluorid von Titan, Niob, Tantal oder Antimon ist.

4. Ionische Flüssigkeit nach Anspruch 3, bei der die Lewis-Säure eine Antimon-Verbindung ist.

5. Ionische Flüssigkeit nach einem der Ansprüche 1 bis 4, bei der das organische Salz X⁺A⁻ ein Chlorid oder ein Fluorid ist und das Kation X⁺ einer der nachstehenden allgemeinen Formeln
RIR2R3R4N⁺
R¹R²R³R⁴P⁺
R¹R²R³S⁺
entspricht, worin die Symbole R¹ bis R⁴, die gleich oder verschieden sind, jeweils eine gesättigte oder ungesättigte, cyclische oder nicht-cyclische oder aromatische Kohlenwasserstoffgruppe, Chlorkohlenwasserstoffgruppe, Fluorkohlenwasserstoffgruppe, Chlorfluorkohlenwasserstoffgruppe oder Fluorkohlenstoffgruppe mit 1 bis 10 Kohlenstoffatomen bedeuten, wobei eine oder mehrere dieser Gruppen ferner ein oder mehrere Heteroatome enthalten können.

6. Ionische Flüssigkeit nach einem der Ansprüche 1 bis 4, bei der das organische Salz X⁺A⁻ Chlorid oder ein Fluorid ist, und das Kation X⁺ einen Teil eines gesättigten oder ungesättigten oder aromatischen Heterozyklus mit 1 bis 3 Stickstoffatomen, Phosphoratomen oder Schwefelatomen bildet und das einer der nachstehenden allgemeinen Formeln 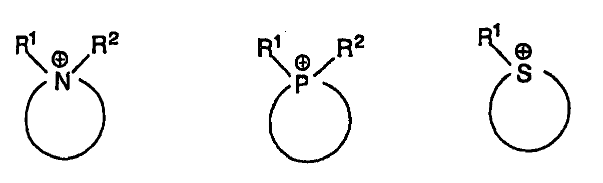 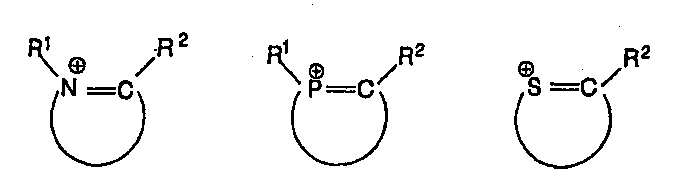 entspricht, in denen er R¹ und R² wie in Anspruch 5 definiert sind.

7. Ionische Flüssigkeit nach Anspruch 5 oder 6, bei der das Kation X⁺ Tetraalkylammonium, Tetraalkylphosphonium, Trialkylsulfonium, Alkylpyridinium, Dialkylimidazolium oder Trialkylimidazolium und vorzugsweise Trimethylsulfonium, N-Ethylpyridinium, N-Butylpyridinium, 1-Ethyl-3-methylimidazolium oder 1-Butyl-3-methylimidazolium bedeutet.

8. Verfahren zur Fluorierung einer gesättigten oder ungesättigten Verbindung mit C-Cl-Bindungen mit HF in flüssiger Phase, **dadurch gekennzeichnet, dass** als Katalysator eine ionische Flüssigkeit nach einem der Ansprüche 1 bis 7 verwendet wird.
